# EUROPÄISCHE PATENTSCHRIFT

(11) **EP 2 546 646 B1**
(45) Veröffentlichungstag und Bekanntmachung des Hinweises auf die Patenterteilung: **16.11.2016**
(21) Anmeldenummer: 11173456.2
(22) Anmeldetag: 11.07.2011
(51) Int. Cl.: G01N 33/28

(54) **Analyse von in Transformatoröl gelösten Gasen**
Analysis of gases dissolved in transformer oil
Analyse des gaz dissous dans l'huile d'un transformateur

(43) Veröffentlichungstag der Anmeldung: 16.01.2013
(73) Patentinhaber: EMH Energie-Messtechnik GmbH, 21438 Brackel (DE)
(72) Erfinder: Riepenhusen, Bernd, DE-21244 Buchholz i.d. Nordheide (DE); Schröder, Karsten, DE-20146 Hamburg (DE)
(74) Vertreter: Seemann & Partner Patentanwälte mbB

(56) Entgegenhaltungen:
- WO-A1-96/36869
- WO-A1-99/53314
- US-A1- 2005 086 998
- US-A1- 2006 249 385
- US-A1- 2009 166 197
- US-B1- 6 436 257
- TSUKIOKA H ET AL: "New Apparatus for Detecting Transformer Faults", IEEE TRANSACTIONS ON ELECTRICAL INSULATION, IEEE, US, Bd. EI-20, Nr. 2, 1. April 1986 (1986-04-01), Seiten 221-229, XP011177581, ISSN: 0018-9367
- TSUKIOKA H ET AL: "New Apparatus for Detecting H2, CO, and CH4 Dissolved in Transformer Oil", IEEE TRANSACTIONS ON ELECTRICAL INSULATION, IEEE, US, Bd. EI-1, Nr. 4, 1. August 1983 (1983-08-01), Seiten 409-419, XP011162710, ISSN: 0018-9367
- Claudius Hummel: "Charakterisierung einer Membran-Gassensor-Kombination zum Nachweis von gelösten Gasen", , 31. August 2001 (2001-08-31), XP55011057, Gefunden im Internet: URL:http://geb.uni-giessen.de/geb/volltext e/2001/496/pdf/d010099.pdf [gefunden am 2011-11-02]

## Beschreibung

Die Erfindung betrifft eine Einrichtung zur, insbesondere quantitativen, Analyse von in einem Transformatoröl gelösten Gasen umfassend eine Gasmesskammer, die mittels einer Membran gegenüber dem Transformatoröl abgedichtet ist, wobei die Membran eine erste Permeabilität für Kohlenstoffmonoxid und eine zweite Permeabilität für Acetylen aufweist, und einen in der Gasmesskammer angeordneten Gassensor, der eine erste Sensitivität für Kohlenstoffmonoxid und eine zweite Sensitivität für Acetylen aufweist, wobei ein in der Gasmesskammer angeordneter Gassensor vorhanden ist, der eine erste Sensitivität für Kohlenstoffmonoxid und eine zweite Sensitivität für Acetylen aufweist, wobei das Verhältnis der zweiten Sensitivität für Acetylen zur ersten Sensitivität für Kohlenstoffmonoxid größer als 1 ist.

Transformatoren gehören zu den wichtigsten und kostenintensivsten Betriebsmitteln in der elektrischen Energieversorgung. Das Auftreten von Fehlern bei diesen Komponenten führt deshalb nicht nur zu Unterbrechungen in der elektrischen Energieversorgung, sondern auch zu wirtschaftlichen Schäden.

Ein Transformatorbetrieb soll eine kontinuierliche Energieversorgung möglichst über Jahre hinweg fehlerfrei sichern. Aus diesem Grund ist es erwünscht, dass auftretende Fehler, die eventuell Ausfälle der Transformatoren verursachen können, rechtzeitig erkannt werden, um geeignete Maßnahmen zur Fehlerbeseitigung einzuleiten.

Bei den häufigsten Fehlern ist die Isolation des Transformators direkt oder indirekt betroffen. Eine kontinuierliche Erfassung des Isolationszustandes kann somit große Fehler verhindern, die Nutzungsdauer eines Transformators verlängern und die Instandhaltung unterstützen.

Als Isoliermittel wird in Leistungstransformatoren eine Kombination aus einem flüssigen, insbesondere mineralölhaltigen, und einem festen, insbesondere cellulosehaltigen, Isolierstoff eingesetzt. Fehler in der Isolation sind fast immer von der Zersetzung der Isolationsmaterialien unter Entwicklung von Gasen begleitet, wobei die Gase wenigstens teilweise im Isolieröl gelöst werden. Die Menge und Zusammensetzung der Zersetzungsgase hängt sowohl von der Isolierflüssigkeit als auch von der Art des zugrundeliegenden Fehlers und der dadurch freigesetzten Energiemenge ab. Mögliche Betriebsfehler sind beispielsweise Teilentladungen und Kreisströme, örtliche Überhitzungen durch Kurzschluss, hohe Übergangswiderstände oder starke Wirbelströme sowie Lichtbogenentladungen und -überschläge. Dabei erfolgt durch elektrische und/oder thermische Energiezufuhr eine Zerstörung der langkettigen Moleküle im Isolieröl, wodurch insbesondere Wasserstoff (H₂) und leichte Kohlenwasserstoffverbindungen, wie z.B. Methan (CH₄), Ethan (C₂H₆), Ethylen (C₂H₄) und Acetylen (C₂H₂), entstehen. Bei der Zersetzung von Cellulose, das als Bestandteil der Feststoffisolation eingesetzt wird, entstehen zusätzlich Kohlenstoffmonoxid (CO) und Kohlenstoffdioxid (CO₂). Diese Zusammenhänge sind bekannt und beispielsweise dokumentiert in IEEE Std C57.104-1991"'IEEE Guide for the Interpretation of Gases Generated in Oil Immersed Transformers", ISBN 1-55937-157-9, Institute of Electrical and Electronics Engineers, Inc., 1992.

Um frühzeitig Hinweise auf Fehlfunktionen des Transformators zu erhalten, wird im Betrieb die Konzentration der im Transformatoröl gelösten Gase überwacht, beispielsweise durch regelmäßige Entnahme von Ölproben mit anschließender Laboranalyse. Es sind auch Analysegeräte bekannt, die am Transformator installiert sind.

Dabei stellt sich das Problem, dass signifikante Fehlfunktionen abhängig von ihrer Ursache durch unterschiedliche Gase nachweisbar sind, wobei die jeweils signifikante Konzentration abhängig von dem relevanten Gas stark variiert. Im Stand der Technik werden daher die Konzentrationen mehrerer Fehlergase bzw. Indikatorgase separat überwacht, wozu entsprechend aufwändige und teure Apparaturen eingesetzt werden. Aus WO 99/53314 A1 ist beispielsweise eine Vorrichtung bekannt, bei der die Konzentrationen von Wasserstoff, Kohlenstoffmonoxid, Acetylen und Ethylen unter Verwendung mehrerer Membranen und Gassensoren separat erfasst werden.

Das Dokument US 6 436 257 B1 offenbart einen Gassensor mit einer Brennstoffzelle zum Nachweis von Acetylen in einer Flüssigkeit, wobei die Brennstoffzelle durch eine Gasextraktionsmembran von der Flüssigkeit getrennt ist.

Das Dokument US 2009/0166197 A offenbart ebenfalls eine Brennstoffzelle zum Nachweis von Gasen in Flüssigkeiten, wobei in der Brennstoffzelle mehrere, für jeweils unterschiedliche Gase optimierte Elektroden vorgesehen sind.

Aus H. Tsukioka et al., "New Apparatus for Detecting Transformator Faults", IEEE Transactions on Electrical Insulation, IEEE, US, Vol. EI-20, No. 2, April 1, 1986, pp. 221-229, ISSN: 0018-9367 ist ein Diagnoseapparat zum Nachweis von in Transformatoröl gelösten Gasen bekannt. Der Diagnoseapparat umfasst eine PFA-Membran, einen Gaschromatographen und einen Gassensor.

Der vorliegenden Erfindung liegt die Aufgabe zugrunde, die Analyse von in einem Transformatoröl gelösten Gasen zu vereinfachen.

Diese Aufgabe wird gelöst durch eine Einrichtung zur, insbesondere quantitativen, Analyse von in einem Transformatoröl gelösten Gasen umfassend eine Gasmesskammer, die mittels einer Membran gegenüber dem Transformatoröl abgedichtet ist, wobei die Membran eine erste Permeabilität für Kohlenstoffmonoxid und eine zweite Permeabilität für Acetylen aufweist, und einen in der Gasmesskammer angeordneten Gassensor, der eine erste Sensitivität für Kohlenstoffmonoxid und eine zweite Sensitivität für Acetylen aufweist, wobei das Verhältnis der zweiten Sensitivität für Acetylen zur ersten Sensitivität für Kohlenstoffmonoxid größer als 1 ist, wobei die Einrichtung dadurch fortgebildet ist, dass eine Messvorrichtung für den Gassensor vorgesehen ist, mittels der eine gewichtete Gesamtgaskonzentration, die abhängig ist von der Konzentration gelösten Kohlenstoffmonoxids im Transformatoröl und von der Konzentration gelösten Acetylens im Transformatoröl, erfasst ist oder wird oder erfassbar ist, wobei die zweite Permeabilität der Membran für Acetylen normiert auf die erste Permeabilität der Membran für Kohlenstoffmonoxid zwischen 1,25 und 5 ist und ein Material der Membran niederdichtes Polyethylen oder Polypropylen oder Perfluorethylen mit Perfluoro-Co-Polymeren ist.

Die, insbesondere als Folie ausgebildete, Membran ist mit einer Seite der Gasmesskammer zugewandt und grenzt mit der anderen Seite entweder direkt an das Transformatoröl oder an einen mit dem Transformatoröl in Kontakt stehenden Gasraum. Die im Transformatoröl gelösten Gase gehen bis zu einer Gleichgewichtskonzentration, die insbesondere für verschiedene Gase unterschiedlich ist, in die gasförmige Phase über, d.h. die gelösten Gase gasen zu einem gewissen Grade aus, und gelangen, insbesondere durch Diffusion, durch die Membran hindurch in die Gasmesskammer. Das Transformatoröl selbst wird dabei durch die Membran zurückgehalten, so dass die Gasmesskammer mittels der Membran gegenüber dem Transformatoröl abgedichtet ist.

Erfindungsgemäß weist die Membran unterschiedliche Permeabilitäten für Kohlenstoffmonoxid und für Acetylen auf. Die Permeabilität stellt dabei eine in erster Linie vom Membranmaterial abhängige Größe dar, die üblicherweise in der SI-Einheit Barr angegeben wird und ein Maß für die Durchlässigkeit einer Membran für ein gegebenes Gas ist. Durch die Erfindung wird somit insbesondere erreicht, dass das Verhältnis der Konzentration von Acetylen zu der Konzentration von Kohlenstoffmonoxid in der Gasmesskammer größer ist als das entsprechende Verhältnis in einem vergleichbaren Gasraum, der in direktem Kontakt, d.h. ohne trennende Membran, mit dem Transformatoröl steht.

Ein in der Gasmesskammer angeordneter Gassensor erfasst die Konzentration der in der Gasmesskammer vorhandenen Gase, wobei der Gassensor erfindungsgemäß jeweils unterschiedliche Sensitivitäten für Kohlenstoffmonoxid und für Acetylen aufweist. Die Sensitivität des Gassensors auf ein bestimmtes Gas bezeichnet dabei das Verhältnis eines mittels des Gassensors bereitgestellten Ausgangssignals und der Konzentration des gegebenen Gases in der Gasmesskammer für den Fall, dass keine weiteren nachweisbaren Gase in der Gasmesskammer vorhanden sind. Als nachweisbar werden in diesem Zusammenhang diejenigen Gase bezeichnet, auf die der Gassensor überhaupt anspricht. Üblicherweise sind Gase wie Stickstoff (N₂) und Sauerstoff (O₂) für Gassensoren zum Nachweis brennbarer Gase nicht nachweisbar.

Der Gassensor stellt ein Ausgangssignal bereit, das mittels einer Messvorrichtung, die insbesondere in den Gassensor integriert sein kann, in ein, insbesondere elektrisches, Messsignal umgewandelt wird oder umwandelbar ist. Bei dem Messsignal handelt es sich beispielsweise um ein analoges oder digitales Signal für eine elektrische Spannung oder einen elektrischen Strom oder eine integrierte bzw. aufsummierte elektrische Ladung.

Der Erfindung liegt die Idee zugrunde, mittels des Gassensors eine gemeinsame Messgröße zu erfassen, die wenigstens von der Kohlenstoffmonoxidkonzentration und von der Acetylenkonzentration im Transformatoröl abhängt und im Rahmen der Erfindung als gewichtete Gesamtgaskonzentration bezeichnet wird. Die gewichtete Gesamtgaskonzentration setzt sich zusammen aus der jeweiligen Gaskonzentration multipliziert mit einem individuellen Gewichtsfaktor, aufsummiert über alle nachweisbaren Gase. Für ein gegebenes Gas hängt der Gewichtsfaktor dabei von der Permeabilität der Membran für das entsprechende Gas und der Sensitivität des Gassensors für das entsprechende Gas ab. Kennzeichnend für die Erfindung ist somit die Kombination einer geeigneten Membran mit einem geeigneten Gassensor.

Durch die erfindungsgemäße Einrichtung mit der beschriebenen Membran und dem angegebenen Gassensor werden die Gewichtsfaktoren von Kohlenstoffmonoxid und von Acetylen dabei derart vorgegeben, dass der Beitrag von Acetylen in der gewichteten Gesamtgaskonzentration im Vergleich zu dem Beitrag von Kohlenstoffmonoxid verstärkt oder betont wird. Obwohl die Konzentration von Acetylen im Transformatoröl bei einer Transformatorfehlfunktion mit Zersetzung des Transformatoröls üblicherweise wesentlich geringer ist als die Konzentration von Kohlenstoffmonoxid bei einer Transformatorfehlfunktion mit Zersetzung der Feststoffisolation und vergleichbarem Schweregrad wird durch die Erfindung erreicht, dass sowohl bei Zersetzung der Feststoffisolation unter Entstehung von Kohlenstoffmonoxid als auch bei Zersetzung des Transformatoröls unter Entstehung von Acetylen ein signifikanter Anstieg der gewichteten Gesamtgaskonzentration zu verzeichnen ist.

Die erfasste gemittelte Gesamtgaskonzentration wird beispielsweise mit einem vorgegebenen oder vorgebbaren Schwellenwert verglichen. Dadurch wird eine besonders einfache quantitative Auswertung der gewichteten Gesamtgaskonzentration bzw. eine besonders einfache quantitative Analyse von in einem Transformatoröl gelösten Gasen ermöglicht.

Wenn die gemittelte Gesamtgaskonzentration wiederholt erfasst bzw. gemessen wird bzw. die Messung insbesondere in regelmäßigen zeitlichen Abständen kontinuierlich ausgeführt wird, wird darüber hinaus ermöglicht, den zeitlichen Verlauf der gewichteten Gesamtgaskonzentration und somit der Konzentrationen von im Transformatoröl gelösten Gase zu überwachen. Insbesondere ist so auch eine Steigung der gewichteten Gesamtgaskonzentration mit der Zeit ermittelbar, beispielsweise als Differenz zweier nacheinander ermittelter bzw. erfasster Messwerte für die gewichtete Gesamtgaskonzentration geteilt durch den zeitlichen Abstand der beiden Messungen. Dadurch wird sehr frühzeitig eine Fehlfunktion des Transformators erkennbar, weil ein schneller Anstieg der gewichteten Gesamtgaskonzentration bzw. eine große Steigung der zeitlich versetzt zueinander erfassten Messwerte bereits auf einen Fehler hindeutet, bevor die absolute Konzentration gelöster Gase und somit die gewichtete Gesamtgaskonzentration signifikant wird.

Vorteilhafterweise ist die zweite Permeabilität der Membran für Acetylen normiert auf die erste Permeabilität der Membran auf Kohlenstoffmonoxid kleiner als 10. Dadurch wird sichergestellt, dass die ermittelte gewichtete Gesamtgaskonzentration ausreichend sensitiv ist auf einen Anstieg der Konzentration von Kohlenstoffmonoxid im Transformatoröl.

Versuche haben ergeben, dass die zweite Permeabilität der Membran für Acetylen normiert auf die erste Permeabilität der Membran für Kohlenstoffmonoxid besonders vorteilhaft zwischen 1,25 und 5, insbesondere zwischen 1,5 und 3,5, ist. Es ist erfindungsgemäß vorgesehen, dass die zweite Permeabilität der Membran für Acetylen normiert auf die erste Permeabilität der Membran für Kohlenstoffmonoxid zwischen 1,25 und 5 ist.

Ferner ist erfindungsgemäß ein Material der Membran niederdichtes Polyethylen (englisch: Low-density Polyethylene, LDPE) oder Polypropylen oder Perfluorethylen (Teflon) mit Perfluoro-Co-Polymeren. Ferner ist bevorzugt vorgesehen, dass ein Material der Membran eine Kombination dieser Materialien ist. Diese Materialien bieten das erfindungsgemäße Verhältnis der Permeabilitäten für Kohlenstoffmonoxid und Acetylen. Darüber hinaus werden sie wenig bis gar nicht von mineralölhaltigen Transformatorölen zersetzt und gewährleisten somit eine lange Lebensdauer und einen störungsfreien Betrieb der erfindungsgemäßen Einrichtung.

Vorteilhaft ist außerdem, wenn die zweite Sensitivität des Gassensors für Acetylen normiert auf die erste Sensitivität des Gassensors für Kohlenstoffmonoxid kleiner als 10 ist, insbesondere zwischen 1,25 und 5, insbesondere zwischen 1,5 und 2,5, beträgt. Hierdurch wird sichergestellt, dass die ermittelte gewichtete Gesamtgaskonzentration ausreichend sensitiv ist auf einen Anstieg der Konzentration von Kohlenstoffmonoxid im Transformatoröl.

Vorzugsweise weist der Gassensor eine dritte Sensitivität für Wasserstoff auf, wobei die dritte Sensitivität des Gassensors für Wasserstoff normiert auf die erste Sensitivität des Gassensors für Kohlenstoffmonoxid kleiner als 1 ist. Die dritte Sensitivität des Gassensors für Wasserstoff normiert auf die erste Sensitivität des Gassensors für Kohlenstoffmonoxid ist außerdem vorteilhafterweise größer als 0,10, insbesondere größer als 0,15.

Hierdurch wird Wasserstoff als drittes wichtiges Fehlergas bzw. Indikatorgas für Fehlfunktionen eines Transformators in die gewichtete Gesamtgaskonzentration mit eingebunden, wobei der Beitrag von Wasserstoff zur gewichteten Gesamtgaskonzentration erfindungsgemäß relativ zum Beitrag von Kohlenstoffmonoxid und entsprechend auch zum Beitrag von Acetylen unterdrückt ist. Dadurch wird berücksichtigt, dass Wasserstoff im Vergleich zu Acetylen erst bei relativ hohen Konzentrationen auf signifikante Fehlfunktionen des Transformators hinweist bzw. schon bei geringen und unbedeutenden Fehlern in signifikanten Mengen entsteht.

Im Rahmen der Erfindung ist es vorteilhaft, wenn der Gassensor als elektrochemischer Gassensor mit einer ersten Elektrode, einer zweiten Elektrode und einem Elektrolyten ausgebildet ist. Bevorzugt ist der Elektrolyt dabei als Festkörperionenleiter ausgebildet, wobei insbesondere die Elektroden als poröse Metallelektroden ausgebildet sind. Derartige Gassensoren sind strukturell einfach aufgebaut, zuverlässig im Betrieb und billig in der Herstellung.

Das Ansprechverhalten derartiger Gassensoren auf verschiedene Gase, d.h. die gassortenabhängige Sensitivität der Gassensoren, ist unter anderem abhängig von den Materialien und der relativen Anordnung der Elektroden und des Elektrolyten. Außerdem wird die Sensitivität auf einzelne Gase beeinflusst durch physikalisch oder chemisch wirkende Filter oder Katalysatoren, die zwischen dem Sensor und dem zu analysierenden Gas oder innerhalb des Gassensors angeordnet sind. Die Sensitivität des Gassensors auf die einzelnen Gase ist durch Anpassung der genannten Einflussfaktoren über weite Bereiche variierbar und wird so geeignet eingestellt.

Bei einer bevorzugten Weiterbildung des Gassensors ist vorgesehen, dass ein Material der ersten Elektrode Silber ist oder die erste Elektrode aus Silber besteht, dass ein Material der zweiten Elektrode Platin ist oder die zweite Elektrode aus Platin besteht, und dass ein Material des Elektrolyten Hydronium-Nasicon ist oder der Elektrolyt aus Hydronium-Nasicon besteht. Dem elektrochemischen Nachweis liegen somit chemische Reaktionen unter Beteiligung von Silber an der ersten Elektrode und unter Beteiligung von Platin an der zweiten Elektrode zugrunde, wobei ein Ionentransport zwischen den Elektroden mittels Nasicon (Abkürzung für Natrium Super Ion Conductor), insbesondere eines Nasicon-Kristalls, erfolgt, wobei die Na⁺-Ionen des Nasicons wenigstens teilweise durch H₃O⁺-Ionen ersetzt sind. Gassensoren mit diesen Materialien weisen bei geeigneter Ausführung eine größere Sensitivität für Acetylen auf als für Kohlenstoffmonoxid.

Eine alternative vorteilhafte Weiterbildung des Gassensors zeichnet sich dadurch aus, dass ein Material der ersten Elektrode Silber ist oder die erste Elektrode aus Silber besteht, dass ein Material der zweiten Elektrode Palladium ist oder die zweite Elektrode aus Palladium besteht und dass ein Material des Elektrolyten Antimon-Pentoxid ist oder der Elektrolyt aus Antimon-Pentoxid besteht. Dem elektrochemischen Nachweis liegen in diesem Fall chemische Reaktionen unter Beteiligung von Silber an der ersten Elektrode und unter Beteiligung von Palladium an der zweiten Elektrode zugrunde, wobei ein Ionentransport zwischen den Elektroden mittels Antimon-Pentoxid, insbesondere mittels eines Antimon-Pentoxid-Kristalls, erfolgt. Gassensoren mit diesen Materialien weisen bei geeigneter Ausführung eine größere Sensitivität für Acetylen auf als für Kohlenstoffmonoxid.

Die beschriebenen Weiterbildungen des Gassensors weisen eigenständigen erfinderischen Gehalt auf, weil die der Erfindung zugrundeliegende Aufgabe auch gelöst wird durch eine Einrichtung zur quantitativen Analyse von in einem Transformatoröl gelösten Gasen umfassend eine Gasmesskammer, die mittels einer Membran gegenüber dem Transformatoröl abgedichtet ist, wobei die Membran eine erste Permeabilität für Kohlenstoffmonoxid und eine zweite Permeabilität für Acetylen aufweist, und einen in der Gasmesskammer angeordneten Gassensor, der eine erste Sensitivität für Kohlenstoffmonoxid und eine zweite Sensitivität für Acetylen aufweist, die dadurch weitergebildet ist, dass der Gassensor als elektrochemischer Gassensor mit einer ersten Elektrode, einer zweiten Elektrode und einem Elektrolyten ausgebildet ist, wobei ein Material der ersten Elektrode Silber ist, ein Material der zweiten Elektrode Platin ist und ein Material des Elektrolyten Hydronium-Nasicon ist oder wobei ein Material der ersten Elektrode Silber ist, ein Material der zweiten Elektrode Palladium ist und ein Material des Elektrolyten Antimon-Pentoxid ist.

Eine Reihe von Weiterbildungen, die auch jeweils eigenständigen erfinderischen Gehalt aufweisen, betreffen die Abmessungen der Membran und der Gasmesskammer, insbesondere die Dicke der Membran, die aktive Fläche der Membran und das Volumen der Gasmesskammer.

Die Membran weist vorzugsweise eine Dicke zwischen 0,01 mm und 0,05 mm, insbesondere zwischen 0,02 mm und 0,04 mm, auf.

Die Membran weist vorzugsweise eine aktive Fläche zwischen 2 cm² und 10 cm², insbesondere zwischen 3 cm² und 7 cm², auf. Die aktive Fläche der Membran bezeichnet dabei denjenigen Anteil der Gesamtfläche, über den der Gasaustausch zwischen dem Transformatoröl und der Gasmesskammer stattfindet. Hierbei bezeichnet die Gesamtfläche die makroskopische Abmessung der Membran und nicht etwa eine mikroskopische Oberfläche oder dergleichen.

Je geringer die Dicke und je größer die aktive Fläche der Membran ist, desto größer ist die Durchflussmenge an Gasen. Andererseits beeinträchtigen eine geringe Dicke und eine große aktive Fläche der Membran die mechanische Stabilität derselben bis hin zur Gefahr von Membranrissen.

Durch die genannten Werte für die Dicke der Membran und die aktive Fläche der Membran wird jeweils sichergestellt, dass die Durchflussmenge an Gasen durch die Membran und die mechanische Stabilität der Membran gleichzeitig optimiert werden.

Im Gegensatz zur Permeabilität, die eine reine Materialeigenschaft beschreibt, hängt die Durchflussmenge, d.h. das pro Zeiteinheit durch die Membran hindurchtretende Gasvolumen bzw. die pro Zeiteinheit durch die Membran hindurchtretende Masse des betreffenden Gases, neben dem Membranmaterial von weiteren Faktoren ab, beispielsweise von der Größe und der Dicke der Membran sowie vom Druckunterschied des Gases auf beiden Seiten der Membran. Eine hohe Durchflussmenge ist von Vorteil, weil dadurch ein schneller Gasaustausch zwischen dem Transformatoröl und der Gasmesskammer ermöglicht wird. Dies erlaubt eine entsprechend kurze Messdauer bzw. eine hohe Wiederholrate an kontinuierlich hintereinander ausgeführten Messungen.

Die Gasmesskammer weist vorzugsweise ein Volumen zwischen 20 cm³ und 100 cm³, insbesondere zwischen 25 cm³ und 50 cm³, auf. Hierdurch wird insbesondere die zeitliche Entwicklung der Konzentration an Gasen in der Gasmesskammer, die maßgeblich den osmotischen Druck aufgrund der Konzentrationsunterschiede beiderseits der Membran bestimmt, beeinflusst. Da der Druckunterschied beiderseits der Membran durch den osmotischen Druck dominiert wird und die Durchflussmenge von dem Druckunterschied abhängt, trägt im Resultat die geeignete Wahl des Volumens der Gasmesskammer zur Optimierung der Durchflussmenge bei.

Die Aufgabe wird außerdem gelöst durch die Verwendung einer erfindungsgemäßen Einrichtung zur, insbesondere quantitativen, Analyse von in einem Transformatoröl gelösten Gasen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: schematisch einen Transformator mit einer erfindungsgemäße Gasanalyseeinrichtung,
- Fig. 2: schematisch eine erfindungsgemäße Gasanalyseeinrichtung und
- Fig. 3: schematisch einen Gassensor einer erfindungsgemäßen Gasanalyseeinrichtung.

In den Zeichnungen sind jeweils gleiche oder gleichartige Elemente und/oder Teile mit denselben Bezugsziffern versehen, so dass von einer erneuten Vorstellung jeweils abgesehen wird.

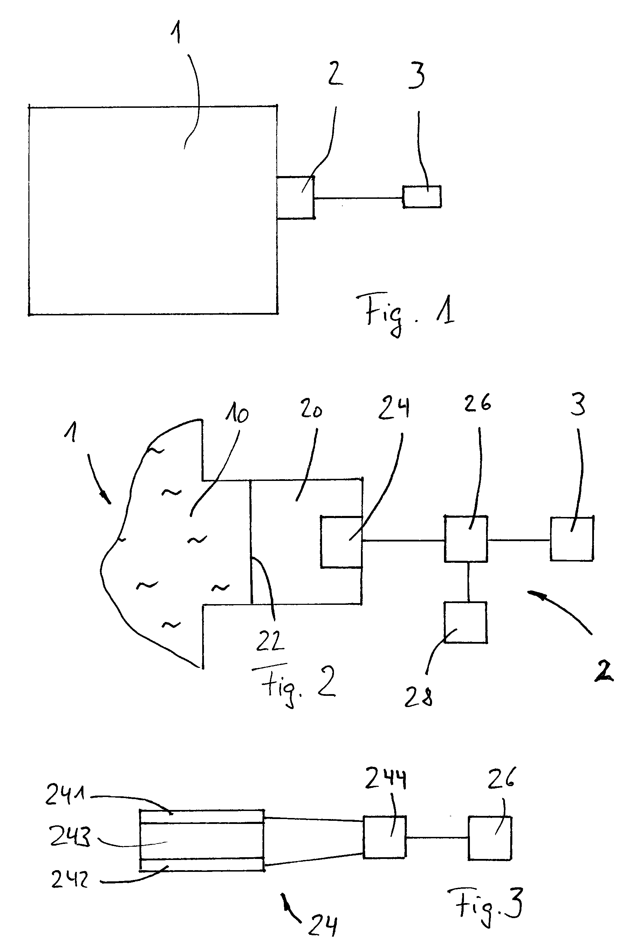

Fig. 1 zeigt schematisch einen Transformator 1. Am Transformator 1 ist eine Gasanalyseeinrichtung 2 angeordnet, die das Transformatoröl 10 des Transformators 1 in regelmäßigen Abständen, beispielsweise alle 20 Minuten, auf gelöste brennbare Gase analysiert eine gewichtete Gesamtgaskonzentration ermittelt. Für den Fall, dass die ermittelte gewichte Gesamtgaskonzentration einen vorgebbaren Grenzwert überschreitet, was gleichbedeutend damit ist, dass die Konzentration wenigstens eines der erfassten Gase im Transformatoröl 10 abnormal hoch ist, wird ein Alarmsignal 3 generiert.

In Fig. 2 ist schematisch ein Ausführungsbeispiel für eine erfindungsgemäße Gasanalyseeinrichtung 2 gezeigt. Die Gasanalyseeinrichtung 2 ist mit dem Transformator 1 verbunden und weist eine Gasmesskammer 20 auf, die mittels einer Membran 22 vom Transformatoröl 10 getrennt bzw. isoliert ist. Die Gasmesskammer 20 hat beispielsweise ein Volumen von etwa 30 cm³ bzw. 30 ml.

Die gezeigte Anordnung Transformatoröl 10-Membran 22-Gasmesskammer 20 ist dabei alternativ durch die Anordnung Transformatoröl 10-Gasraum-Membran 22-Gasmesskammer 22 ersetzbar, wobei unter Gasraum insbesondere ein unmittelbar oberhalb des Transformatoröls 10 angeordnetes und von diesem nicht abgetrenntes Gasvolumen verstanden wird.

Die Membran 22 ist als gasdurchlässige Membran, insbesondere semipermeable Membran, ausgebildet, wobei die Membran 22 unterschiedliche Permeabilitäten für Wasserstoff (H₂), Kohlenstoffmonoxid (CO) und Acetylen (C₂H₂) aufweist. Insbesondere ist die Membran derart gewählt, dass die Permeabilität für Acetylen größer ist als die Permeabilität für Kohlenstoffmonoxid und besteht beispielsweise aus Polypropylen, niederdichtem Polyethylen oder Perfluorethylen mit Perfluoro-Co-Polymeren.

Beispielsweise wird von der Goodfellow GmbH, 61213 Bad Nauheim, Deutschland, eine Membran 22 aus Polyethylen niedriger Dichte mit einer Dicke von 0,025 mm angeboten. Für diese Membran 22 beträgt das Verhältnis der Permeabilität für Acetylen zu der Permeabilität für Kohlenstoffmonoxid etwa 1,5.

Die Membran 22 ist beispielsweise rund mit einem Durchmesser von 40 mm, wobei die aktive Fläche in der erfindungsgemäßen Gasanalyseeinrichtung 2 beispielsweise 5 cm² beträgt. Der Unterschied zwischen aktiver Fläche und Gesamtfläche der Membran 22 ergibt sich dabei insbesondere durch den Einbau der Membran 22, beispielsweise durch das teilweise Abdecken mit einer nicht dargestellten Dichtung am Rand der Membran 22.

Durch die Membran 22 gelangen im Transformatoröl 10 gelöste Gase in die Gasmesskammer 20. Dies gilt im Prinzip für alle Gase, für die die verwendete Membran 22 permeabel ist. Für die beispielhaft genannten Materialien sind dies neben Wasserstoff, Kohlenstoffmonoxid und Acetylen unter anderem auch Methan (CH₄), Ethylen (C₂H₄) und Ethan (C₂H₆). Da die Membran 22 für jedes dieser Gase eine unterschiedliche Permeabilität aufweist, die Gase insbesondere unterschiedlich gut durch die Membran 22 diffundieren, verschieben sich verglichen mit einem Gasraum, der ohne trennende Membran in direktem Kontakt zum Transformatoröl 10 steht, die Konzentrationen der Gase in der Gasmesskammer 20 relativ zueinander. Erfindungsgemäß wird die Membran 22 derart gewählt, dass die Konzentration von Acetylen relativ zur Konzentration von Kohlenstoffmonoxid verstärkt oder betont wird bzw. dass die Konzentration von Kohlenstoffmonoxid relativ zur Konzentration von Acetylen verringert oder gedämpft wird.

In der Gasmesskammer 20 ist ein Gassensor 24 angeordnet, der als elektrochemischer Gassensor ausgebildet ist. In Fig. 3 ist ein schematischer Aufbau eines derartigen Sensors gezeigt.

Der Sensor umfasst zwei Elektroden 241, 242, die über einen Elektrolyten 243 verbunden sind. Nachzuweisendes Gas wird an einer der Elektroden 241, 242 oxidiert. Die entstehenden Ionen wandern durch den Elektrolyten 243 zur anderen Elektrode 242, 241, während die in der Oxidationsreaktion entstehenden Elektronen eine elektrische Potenzialdifferenz zwischen den beiden Elektroden 241, 242 verursachen. Die Elektroden 241, 242 sind über eine Messvorrichtung 244 elektrisch miteinander verbunden, so dass die Elektronen auf der einen Elektrode 241, 242 aufgrund der Potenzialdifferenz über einen geschlossenen elektrischen Kreis innerhalb der Messvorrichtung 244 zur anderen Elektrode 242, 241 fließen. An der zweiten Elektrode 241, 242 findet dann eine chemische Reduktionsreaktion unter Beteiligung der durch den Elektrolyten 243 gewanderten Ionen und der über die Messvorrichtung 244 geflossenen Elektronen statt. Im Ergebnis wird das reagierende Gas chemisch umgewandelt und dadurch verbraucht.

Das durch die Elektronen hervorgerufene elektrische Signal, beispielsweise eine elektrische Spannung, ein elektrischer Strom oder eine integrierte elektrische Ladung, das mittels der Messvorrichtung 244 registriert wird, ist dabei ein Maß für die Konzentration des reagierenden Gases.

Das Verhältnis von Gaskonzentration und Signalstärke variiert dabei von Gas zu Gas, d.h. der Gassensor ist auf unterschiedliche Gase unterschiedlich sensitiv, wobei das Verhältnis bzw. die Sensitivität für ein gegebenes Gas unter anderem abhängig ist von dem Material der Elektroden 241, 242, dem Material des Elektrolyten 243, der geometrischen Anordnung von Elektroden 241, 242 und Elektrolyten 243 sowie der Verwendung chemischer oder physikalischer Filter oder Katalysatoren innerhalb oder außerhalb des Sensors. Dadurch werden die beschriebenen chemischen und physikalischen Reaktionen und Vorgänge abhängig vom reagierenden Gas beeinflusst und so die Sensitivität des Gassensors 24 auf die verschiedenen Gase verändert bzw. geeignet angepasst.

Beispielsweise wird von der City Technology Limited, Portsmouth, UK, unter der Bezeichnung "Sixth Sense Eco-Sure" ein elektrochemischer Kohlenstoffmonoxidsensor 24 angeboten, der eine Querempfindlichkeit bzw. Sensitivität für Acetylen aufweist, die etwa doppelt so groß ist wie die Sensitivität für Kohlenstoffmonoxid.

Im Ergebnis wird mittels des Gassensors 24 ein elektrisches Signal generiert, das einer gewichteten Gesamtgaskonzentration innerhalb der Gasmesskammer 20 entspricht bzw. hierzu proportional ist. Die gewichtete Gesamtgaskonzentration ist dabei definiert als das Produkt der Konzentration eines Gases im Transformatoröl 10 multipliziert mit einem Gewichtsfaktor, aufsummiert über alle beteiligten bzw. nachweisbaren Gase. Die individuellen bzw. gasabhängigen Gewichtsfaktoren hängen dabei unter anderem ab von der Permeabilität der Membran 22 für das entsprechende Gas sowie der Sensitivität des Gassensors 24 für das entsprechende Gas und sind daher durch geeignete Wahl der Membran 22 und des Gassensors 24 relativ zueinander vorgebbar.

Die ermittelte, gewichtete Gesamtgaskonzentration wird in einer Auswertevorrichtung 26 mit einem vorgebbaren Schwellenwert verglichen. Der Schwellenwert wird beispielsweise über eine mit der Auswertevorrichtung 26 verbundene Bedienvorrichtung 28 bereitgestellt. Für den Fall, dass die gewichtete Gesamtgaskonzentration oberhalb des Schwellenwertes liegt, wird ein Alarmsignal 3 generiert. Dieses Alarmsignal 3 wird beispielsweise an die Bedienvorrichtung 28 weitergeleitet und dort in akustischer oder optischer Form dem Bedienpersonal angezeigt.

Ohne Einschränkung der Erfindung sind ausdrücklich auch solche Ausführungsformen umfasst, bei denen die Auswertevorrichtung 26 Bestandteil der Messvorrichtung 244 oder die Messvorrichtung 244 Bestandteil der Auswertevorrichtung 26 ist.

Es ist auch denkbar, das Alarmsignal 3 an eine nicht dargestellte automatische Betriebsführung des Transformators 1 weiterzuleiten.

Bei einer nicht dargestellten Weiterbildung sind in der Auswertevorrichtung 26 mehrere Schwellenwerte vorgesehen, so dass abgestuft nach der ermittelten gewichteten Gesamtgaskonzentration und entsprechend dem Schweregrad der vorliegenden Transformatorfehlfunktion oder Transformatorstörung verschiedene Alarmsignale erzeugt werden.

Die Auswertevorrichtung 26 umfasst in geeigneten Weiterbildungen eine nicht dargestellte Speichervorrichtung zur Aufzeichnung der mittels des Gassensors 24 erfassten Messwerte. Dadurch wird eine zeitlich differenzierte Auswertung der Messwerte für die gewichtete Gesamtgaskonzentration ermöglicht, so dass Anstiege der Messwerte, die auf einen Anstieg der Konzentration gelöster Gase im Transformatoröl 10 und somit auf eine Fehlfunktion des Transformators hindeuten, frühzeitig erkennbar sind. Insbesondere ist es dadurch ermöglicht, bei einem raschen Anstieg der Gaskonzentration bzw. bei einer großen Steigung der Messwerte mit der Zeit ein Alarmsignal zu generieren, selbst wenn die momentanen Werte für die gewichtete Gesamtgaskonzentration (noch) nicht den vorgegebenen Schwellenwert übersteigen. Beispielsweise ist dafür in der Auswertevorrichtung 26 ein weiterer vorgebbarer Schwellenwert für die Steigung der gewichteten Gesamtgaskonzentration mit der Zeit bzw. für die zeitliche Ableitung der Messwerte für die gewichtete Gesamtgaskonzentration vorgesehen, wobei bei Überschreiten dieses Schwellenwertes ein Alarmsignal 3 generiert wird.

### Bezugszeichenliste

- 1: Transformator
- 2: Gasanalyseeinrichtung
- 3: Alarmsignal
- 10: Transformatoröl
- 20: Gasmesskammer
- 22: Membran
- 24: Gassensor
- 26: Auswertevorrichtung
- 28: Bedienvorrichtung
- 241: Elektrode
- 242: Elektrode
- 243: Elektrolyt
- 244: Messvorrichtung

## Patentansprüche

1. Einrichtung (2) zur, insbesondere quantitativen, Analyse von in einem Transformatoröl (10) gelösten Gasen umfassend eine Gasmesskammer (20), die mittels einer Membran (22) gegenüber dem Transformatoröl (10) abgedichtet ist, wobei die Membran (22) eine erste Permeabilität für Kohlenstoffmonoxid und eine zweite Permeabilität für Acetylen aufweist, und einen in der Gasmesskammer (20) angeordneten Gassensor (24), der eine erste Sensitivität für Kohlenstoffmonoxid und eine zweite Sensitivität für Acetylen aufweist, wobei das Verhältnis der zweiten Sensitivität für Acetylen zur ersten Sensitivität für Kohlenstoffmonoxid größer als 1 ist, **dadurch gekennzeichnet, dass** eine Messvorrichtung (244) für den Gassensor (24) vorgesehen ist, mittels der eine gewichtete Gesamtgaskonzentration, die abhängig ist von der Konzentration gelösten Kohlenstoffmonoxids im Transformatoröl (10) und von der Konzentration gelösten Acetylens im Transformatoröl (10), erfasst ist oder wird oder erfassbar ist, wobei die zweite Permeabilität der Membran (22) für Acetylen normiert auf die erste Permeabilität der Membran (22) für Kohlenstoffmonoxid zwischen 1,25 und 5 ist und ein Material der Membran (22) niederdichtes Polyethylen oder Polypropylen oder Perfluorethylen mit Perfluoro-Co-Polymeren ist.

2. Einrichtung (2) nach Anspruch 1, **dadurch gekennzeichnet, dass** die zweite Permeabilität der Membran (22) für Acetylen normiert auf die erste Permeabilität der Membran (22) auf Kohlenstoffmonoxid kleiner als 10 ist.

3. Einrichtung (2) nach Anspruch 1 oder 2, **dadurch gekennzeichnet, dass** die zweite Permeabilität der Membran (22) für Acetylen normiert auf die erste Permeabilität der Membran (22) für Kohlenstoffmonoxid zwischen 1,5 und 3,5, ist.

4. Einrichtung (2) nach einem der Ansprüche 1 bis 3, **dadurch gekennzeichnet, dass** die zweite Sensitivität des Gassensors (24) für Acetylen normiert auf die erste Sensitivität des Gassensors (24) für Kohlenstoffmonoxid kleiner als 10 ist.

5. Einrichtung (2) nach einem der Ansprüche 1 bis 4, **dadurch gekennzeichnet, dass** die zweite Sensitivität des Gassensors (24) für Acetylen normiert auf die erste Sensitivität des Gassensors für Kohlenstoffmonoxid zwischen 1,25 und 5, insbesondere zwischen 1,5 und 2,5, ist.

6. Einrichtung (2) nach einem der Ansprüche 1 bis 5, **dadurch gekennzeichnet, dass** der Gassensor (24) eine dritte Sensitivität für Wasserstoff aufweist, wobei die dritte Sensitivität des Gassensors (24) für Wasserstoff normiert auf die erste Sensitivität des Gassensors (24) für Kohlenstoffmonoxid kleiner als 1 ist.

7. Einrichtung (2) nach Anspruch 6, **dadurch gekennzeichnet, dass** die dritte Sensitivität des Gassensors (24) für Wasserstoff normiert auf die erste Sensitivität des Gassensors (24) für Kohlenstoffmonoxid größer als 0,10, insbesondere größer als 0,15, ist.

8. Einrichtung (2) nach einem der Ansprüche 1 bis 7, **dadurch gekennzeichnet, dass** der Gassensor (24) als elektrochemischer Gassensor (24) mit einer ersten Elektrode (241, 242), einer zweiten Elektrode (242, 241) und einem Elektrolyten (243) ausgebildet ist.

9. Einrichtung nach Anspruch 8, **dadurch gekennzeichnet, dass** der Elektrolyt (243) als Festkörperionenleiter ausgebildet ist, wobei insbesondere die Elektroden (241, 242) als poröse Metallelektroden ausgebildet sind.

10. Einrichtung nach Anspruch 8 oder 9, **dadurch gekennzeichnet, dass** ein Material der ersten Elektrode (241, 242) Silber ist, dass ein Material der zweiten Elektrode (242, 241) Platin ist und dass ein Material des Elektrolyten (243) Hydronium-Nasicon ist.

11. Einrichtung nach Anspruch 8 oder 9, **dadurch gekennzeichnet, dass** ein Material der ersten Elektrode (241, 242) Silber ist, dass ein Material der zweiten Elektrode (242, 241) Palladium ist und dass ein Material des Elektrolyten (243) Antimon-Pentoxid ist.

12. Einrichtung (2) nach einem der Ansprüche 1 bis 11, **dadurch gekennzeichnet, dass** die Membran (22) eine Dicke zwischen 0,01 mm und 0,05 mm, insbesondere zwischen 0,02 mm und 0,04 mm, aufweist.

13. Einrichtung (2) nach einem der Ansprüche 1 bis 12, **dadurch gekennzeichnet, dass** die Membran (22) eine aktive Fläche zwischen 2 cm² und 10 cm², insbesondere zwischen 3 cm² und 7 cm², aufweist.

14. Einrichtung (2) nach einem der Ansprüche 1 bis 13, **dadurch gekennzeichnet, dass** die Gasmesskammer (20) ein Volumen zwischen 20 cm³ und 100 cm³, insbesondere zwischen 25 cm³ und 50 cm³, aufweist.

15. Verwendung einer Einrichtung (2) nach einem der Ansprüche 1 bis 14 zur, insbesondere quantitativen, Analyse von in einem Transformatoröl (10) gelösten Gasen.

## Claims

1. A device (2) for in particular the quantitative analysis of gases dissolved in a transformer oil (10), comprising a gas measuring chamber (20) which is sealed by means of a membrane (22) against the transformer oil (10), wherein the membrane (22) has a first permeability for carbon monoxide and a second permeability for acetylene, and a gas sensor (24) that is arranged in the gas measuring chamber (20) and has a first sensitivity to carbon monoxide and a second sensitivity to acetylene, wherein the ratio of the second sensitivity to acetylene and the first sensitivity to carbon monoxide is greater than 1, **characterized in that** a measuring apparatus (244) is provided for the gas sensor (24) by means of which a weighted overall gas concentration which is dependent on the concentration of dissolved carbon monoxide in the transformer oil (10) and on the concentration of the dissolved acetylene in the transformer oil (10) is detected or detectable, wherein the second permeability of the membrane (22) to acetylene standardized to the first permeability of the membrane (22) to carbon monoxide is between 1,25 and 5, and a material of the membrane (22) is low density polyethylene, or polypropylene, or perfluorethylene with perfluoro copolymers.

2. The device (2) according to claim 1, **characterized in that** the second permeability of the membrane (22) to acetylene standardized to the first permeability of the membrane (22) to carbon monoxide is less than 10.

3. The device (2) according to claim 1 or 2, **characterized in that** the second permeability of the membrane (22) to acetylene standardized to the first permeability of the membrane (22) to carbon monoxide is between 1,5 and 3,5.

4. The device (2) according to one of claims 1 to 3, **characterized in that** the second sensitivity of the gas sensor (24) to acetylene standardized to the first sensitivity of the gas sensor (24) to carbon monoxide is less than 10.

5. The device (2) according to one of claims 1 to 4, **characterized in that** the second sensitivity of the gas sensor (24) to acetylene standardized to the first sensitivity of the gas sensor to carbon monoxide is between 1,25 and 5, in particular between 1,5 and 2,5.

6. The device (2) according to one of claims 1 to 5, **characterized in that** the gas sensor (24) has a third sensitivity to hydrogen, wherein the third sensitivity of the gas sensor (24) to hydrogen standardized to the first sensitivity of the gas sensor (24) to carbon monoxide is less than 1.

7. The device (2) according to claim 6, **characterized in that** the third sensitivity of the gas sensor (24) to hydrogen standardized to the first sensitivity of the gas sensor (24) to carbon monoxide is greater than 0,10, in particular greater than 0,15.

8. The device (2) according to one of claims 1 to 7, **characterized in that** the gas sensor (24) is designed as an electrochemical gas sensor (24) with a first electrode (241, 242), a second electrode (242, 241), and an electrolyte (243).

9. The device according to claim 8, **characterized in that** the electrolyte (243) is designed as a solid-state ion conductor, wherein in particular the electrodes (241, 242) are designed as porous metal electrodes.

10. The device according to claim 8 or 9, **characterized in that** a material of the first electrode (241, 242) is silver, a material of the second electrode (242, 241) is platinum, and a material of the electrolyte (243) is hydronium NASICON.

11. The device according to claim 8 or 9, **characterized in that** a material of the first electrode (241, 242) is silver, a material of the second electrode (242, 241) is palladium, and a material of the electrolyte (243) is antimony pentoxide.

12. The device (2) according to one of claims 1 to 11, **characterized in that** the membrane (22) has a thickness between 0,01 mm and 0,05 mm, in particular between 0,02 mm and 0,04 mm.

13. The device (2) according to one of claims 1 to 12, **characterized in that** the membrane (22) has an active surface between 2 cm² and 10 cm², in particular between 3 cm² and 7 cm².

14. The device (2) according to one of claims 1 to 13, **characterized in that** the gas measuring chamber (20) has a volume between 20 m³ and 100 m³, in particular between 25 m³ and 50 m³.

15. Use of a device (2) according to one of claims 1 to 14 for, in particular, the quantitative analysis of gases dissolved in a transformer oil (10).

## Revendications

1. Dispositif (2) pour l'analyse, en particulier quantitative, des gaz dissous dans une huile de transformateur (10), comprenant une chambre de mesure de gaz (20) qui est rendue étanche à l'huile du transformateur (10) par une membrane (22), ladite membrane (22) présentant une première perméabilité au monoxyde de carbone et une deuxième perméabilité à l'acétylène, et un capteur de gaz (24) est disposé dans la chambre de mesure de gaz (20) ayant une première sensibilité au monoxyde de carbone et une deuxième sensibilité à l'acétylène, le rapport de la deuxième sensibilité à l'acétylène à la première sensibilité au monoxyde de carbone étant supérieur à 1, **caractérisé en ce qu'**un dispositif de mesure (244) du capteur de gaz (24) est prévu au moyen d'une concentration totale de gaz pondérée dont la détection dépend de la concentration de monoxyde de carbone dissous dans l'huile de transformateur (10) et de la concentration de l'acétylène dissous dans l'huile de transformateur (10), dans lequel la deuxième perméabilité de la membrane (22) à l'acétylène, normalisée par rapport à la première perméabilité de la membrane (22) au monoxyde de carbone, est comprise entre 1,25 et 5, et un composant de la membrane (22) est du polyéthylène à basse densité ou du polypropylène ou du perfluoro-éthylène avec des copolymère perfluorés.

2. Dispositif (2) selon la revendication 1, **caractérisé en ce que** la deuxième perméabilité de la membrane (22) à l'acétylène, normalisée par rapport à la première perméabilité de la membrane (22) au monoxyde de carbone, est inférieure à 10.

3. Dispositif (2) selon la revendication 1 ou la revendication 2, **caractérisé en ce que** la deuxième perméabilité de la membrane (22) à l'acétylène, normalisée par rapport à la première perméabilité de la membrane (22) au monoxyde de carbone, est comprise entre 1,5 et 3,5.

4. Dispositif (2) selon l'une quelconque des revendications 1 à 3, **caractérisé en ce que** la deuxième sensibilité du capteur de gaz (24) à l'acétylène, normalisée par rapport à la sensibilité du premier capteur de gaz (24) au monoxyde de carbone, est plus inférieure à 10.

5. Dispositif (2) selon l'une quelconque des revendications 1 à 4, **caractérisé en ce que** la deuxième sensibilité du capteur de gaz (24) à l'acétylène, normalisée par rapport à la première sensibilité du détecteur de gaz au monoxyde de carbone, est comprise entre 1,25 et 5, en particulier entre 1,5 et 2,5.

6. Dispositif (2) selon l'une quelconque des revendications 1 à 5, **caractérisé en ce que** le capteur de gaz (24) comprend une troisième sensibilité à l'hydrogène, la troisième sensibilité du capteur de gaz (24) à l'hydrogène, normalisée par rapport à la première sensibilité du capteur de gaz (24) au monoxyde de carbone, étant inférieur à 1.

7. Appareil (2) selon la revendication 6, **caractérisé en ce que** la troisième sensibilité du capteur de gaz (24) à l'hydrogène, normalisée par rapport à la première sensibilité du capteur de gaz (24) au monoxyde de carbone, est supérieure à 0,10, en particulier supérieure à 0,15.

8. Dispositif (2) selon l'une quelconque des revendications 1 à 7, **caractérisé en ce que** le capteur de gaz (24) est sous la forme d'un capteur de gaz électrochimique (24) comprenant une première électrode (241, 242), une deuxième électrode (242, 241) et un électrolyte (243).

9. Dispositif selon la revendication 8, **caractérisé en ce que** l'électrolyte (243) est réalisé sous la forme d'un conducteur ionique solide, en particulier, les électrodes (241, 242) sont sous la forme d'électrodes métalliques poreuses.

10. Dispositif selon la revendication 8 ou la revendication 9, **caractérisé en ce qu'**un matériau de la première électrode (241, 242) est l'argent, un matériau de la deuxième électrode (242, 241) est le platine et un matériau de l'électrolyte (243) est l'hydronium Nasicon.

11. Dispositif selon la revendication 8 ou la revendication 9, **caractérisé en ce qu'**un matériau de la première électrode (241, 242) est l'argent, un matériau de la deuxième électrode (242, 241) est le palladium et un matériau de l'électrolyte (243) est le pentoxyde d'antimoine.

12. Dispositif (2) selon l'une quelconque des revendications 1 à 11, **caractérisé en ce que** la membrane (22) présente une épaisseur comprise entre 0,01 mm et 0,05 mm, en particulier entre 0,02 mm et 0,04 mm.

13. Dispositif (2) selon l'une quelconque des revendications 1 à 12, **caractérisé en ce que** la membrane (22) présente une surface active comprise entre 2 cm² et 10 cm², en particulier entre 3 cm² et 7 cm².

14. Dispositif (2) selon l'une quelconque des revendications 1 à 13, **caractérisé en ce que** la chambre de mesure du gaz (20) a un volume compris entre 20 cm³ et 100 cm³, en particulier entre 25 cm³ et 50 cm³.

15. Utilisation d'un dispositif (2) selon l'une quelconque des revendications 1 à 14 pour l'analyse, en particulier quantitative, des gaz dissous dans une huile de transformateur (10).
